# EUROPEAN PATENT APPLICATION

(11) **EP 3 932 322 A1**
(43) Date of publication of application: **05.01.2022**
(21) Application number: 20763307.4
(22) Date of filing: 28.02.2020
(51) Int. Cl.: A61B 8/00

(54) **IMAGE PROCESSING DEVICE AND CONTROL PROGRAM OF IMAGE PROCESSING DEVICE**

(30) Priority: 28.02.2019 JP 2019036731
(71) Applicant: Lily Medtech Inc., Tokyo 113-0033 (JP)
(72) Inventor: YUGE, Kazuo, Tokyo 113-0033 (JP); OTSU, Teruyuki, Tokyo 113-0033 (JP); UJIIE, Hiroki, Hamura-shi, Tokyo 205-0002 (JP)
(74) Representative: Wallin, Nicholas James
(86) International application number: PCT/JP2020/008290
(87) International publication number: WO 2020/175668

(57) **Abstract**

This image processing device 1 includes a bathtub 10 which is internally filled with a propagating liquid W and in which a test object S is immersed, a measurement device 20 which comprises an element group for irradiating radiation waves into the bathtub 10 and receiving scattered radiation waves, an upper drainage detection sensor 33 which detects drainage of the propagating liquid W from the top of the bathtub 10 to outside of the bathtub 10, and a water level control module 123 which controls the water level of the propagating liquid W in the bathtub 10, wherein the water level control module 123 controls the amount of the propagating liquid W in the bathtub 10 such that before the test object S is immersed in the bathtub 10, the water level is below the top position of the bathtub 10, and performs control such that after the test object S has been immersed in the bathtub 10, the propagating liquid W is supplied into the bathtub 10 at least until drainage of the propagating liquid W is detected by the upper drainage detection sensor 33. By this means, in an automatic scan-type image processing device, it is possible with a simple configuration to image a test object with no omissions.

## Description

### Technical Field

The present invention relates to an image processing apparatus and an image processing apparatus control program.

### Background Art

As methods for measuring various parts of the body of an examinee, medical image processing apparatuses (modalities) have come into widespread use, typical examples of which include an ultrasound diagnostic apparatus, an X-ray diagnostic apparatus, computed tomography (CT), and magnetic resonance imaging (MRI). Out of these image processing apparatuses, for example, an ultrasound diagnostic apparatus causes an ultrasound probe to emit ultrasound into the inside of the test object and receive ultrasound reflection waves (echoes) generated by differences in the acoustic impedance of the tissue of the test object. Furthermore, an ultrasound tomographic image representing the structure of the internal tissue of the test object is generated on the basis of an electric signal obtained through this reception, and the image is displayed on, for example, a monitor. Ultrasound diagnostic apparatuses are widely used in making a morphological diagnosis since these apparatuses are less invasive to a test object and can be used to observe the state of in vivo tissue in real time using tomographic images and the like.

To find a disease such as breast cancer, an automatic scanning image processing apparatus that can scan the entirety of a breast, as a test object, has been proposed in recent years. For example, PTLs 1 and 2 disclose a method in which a bath is installed on a measurement table, the inside of the bath is filled with a medium through which radiation waves are able to readily propagate (hereinafter referred to as "propagation liquid"), ultrasonic elements are installed in the inside of the bath, and a coronal image is captured by moving the ultrasonic elements in a direction perpendicular to the bottom surface of the bath to scan a breast positioned so as to hang down into the inside of the bath.

### Citation List

### Patent Literature

PTL 1: Japanese Unexamined Patent Application Publication No. 2013-519455
PTL 2: Japanese Unexamined Patent Application Publication No. 2015-205041

### SUMMARY OF INVENTION

### Technical Problem

It is known that breast cancer originates from inside the mammary glands. Thus, in order to find breast cancer, it is important to set the imaging range so as to include not only the entirety of a breast but also a basal portion of the breast, which is especially a region extending to a border portion between the mammary glands and the pectoralis major, and a part of an axillary region so as to make a thorough diagnosis. In an automatic scanning image processing apparatus, in a case where imaging is performed using ultrasound or radiation waves such as acoustic waves, a region that is not immersed in a propagation liquid (in other words, a region where a layer of air exists midway along a radiation path of a radiation wave) cannot be accurately imaged because the radiation wave attenuates or the like, and thus it is desired that a test object be completely immersed in the propagation liquid.

Here, as described in PTL 1, even in a case of an image processing apparatus in which a test object is (directly) immersed from a top portion of the bath, and imaging is performed, as described above, it is necessary to adjust a liquid level such that the propagation liquid in the bath reaches the basal portion of the test object in a state in which the test object is immersed. However, in the state in which the test object is immersed, the bath is covered by the body of the examinee, and thus it is difficult to visually check the bath. As a result, to optimally adjust the liquid level of the bath without causing the propagation liquid to overflow to the measurement table side, for example, a sensor that can accurately measure the liquid level is needed, and thus the configuration becomes complicated.

Moreover, as described in PTL 2, if a structure is used in which a test object is not immersed in the bath, that is, a structure is used that has a member for holding the test object (a test-subject holding unit 11) and interposed between the bath and the test object, this member prevents the propagation liquid in the bath from entering the measurement table, and thus liquid level adjustment can be achieved with a relatively simple structure. However, if this member that holds the test object is used, an image of the test object is captured in a state in which the test object is held and deformed by this member, and thus a diagnosis needs to be made by considering this deformation, and diagnostic accuracy is likely to vary depending on the skill of the operator (for example, a laboratory technician or another medical worker) who operates the apparatus or the level of interpretation of the doctor in charge. Moreover, the degree of deformation of the test object will differ every time imaging is performed, and thus the shape of the test object will differ every time an examination is performed. Therefore, it is difficult to make a diagnosis based on changes over time. Furthermore, a propagation liquid (an acoustic matching material 12) different from the one in the bath needs to be separately loaded into the region between this member that holds the test object and the test object, and thus the amount of the propagation liquid to be loaded needs to be adjusted separately from liquid level control of the propagation liquid in the bath and every time the test object is changed. Thus, the procedure that is carried out before imaging is significantly complicated because there are many elements to be adjusted. Moreover, sound reflected by the front side and the back side of the test-subject holding unit 11 lowers the imaging performance, and this is another reason why it is difficult to choose this method.

An objective of the present invention is to provide an image processing apparatus that is of an automatic scanning type and capable of thoroughly imaging a test object with a simple configuration, and a control program for the same.

### Solution to Problem

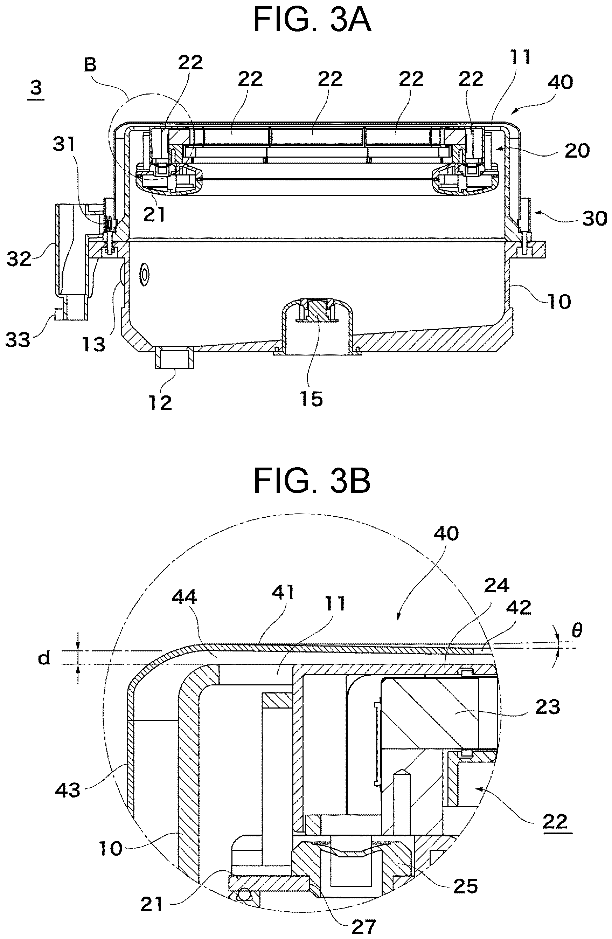

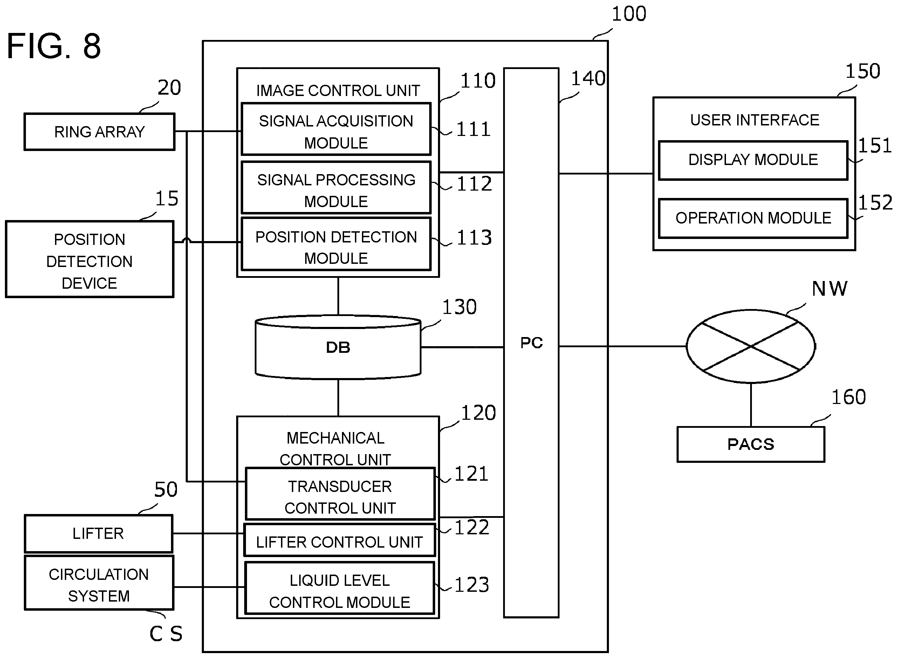

In order to achieve the objective described above, for example, as illustrated in Fig. 3, an image processing apparatus according to a first mode of the present invention includes a bath 10 an inside of which is filled with a propagation liquid W and in which a test object S is to be immersed; a measurement device 20 having a group of elements that irradiates the inside of the bath 10 with a radiation wave and receives the radiation wave, which is a scattered wave; an upper drainage detection sensor 33 that detects draining of the propagation liquid W from a top portion of the bath 10 to outside the bath 10; and a liquid level control module 123 (see Fig. 8.) that controls a liquid level of the propagation liquid W in the inside of the bath 10, and the liquid level control module 123 controls, before the test object S is immersed in the inside of the bath 10, an amount of the propagation liquid W in the inside of the bath 10 such that the liquid level becomes lower than a top end position of the bath 10, and performs control, after the test object S is immersed in the inside of the bath 10, such that the liquid level is increased by supplying the propagation liquid W into the inside of the bath 10 at least until the upper drainage detection sensor 33 detects draining of the propagation liquid W.

With the configuration as described above, the liquid level in the bath can be made to reach the highest liquid level only by performing simple control, and while suppressing overflowing of the propagation liquid onto the examinee side, the propagation liquid can be made to reach the basal portion of the test object. Thus, the test object can be thoroughly imaged by the measurement device.

An image processing apparatus according to a second mode of the present invention is, for example, as illustrated in Fig. 3, the image processing apparatus according to the first mode of the present invention further including a collection tank 30 that is disposed at or near an outer periphery of the bath 10 and collects the propagation liquid W drained from the top portion of the bath 10 to outside the bath 10, and the upper drainage detection sensor 33 is disposed at the collection tank 30.

With the configuration as described above, the propagation liquid that has drained from the top portion of the bath 10 can be assuredly collected, liquid leakage around the bath does not occur, and the propagation liquid can be reused.

An image processing apparatus according to a third mode of the present invention is, for example, as illustrated in Fig. 3, the image processing apparatus according to the first or second mode of the present invention in which a top cover 40 having, at a center portion thereof, an opening 42 through which the test object is insertable is disposed above the bath 10, and a predetermined gap 44 is formed between a top end portion of the bath 10 and an undersurface of the top cover 40.

With the configuration as described above, the propagation liquid flows out through the gap between the top cover and the bath, and thus the propagation liquid does not overflow onto the side where the examinee is lying on the top cover, and consequently liquid level adjustment and measurement can be executed without causing discomfort to the examinee.

An image processing apparatus according to a fourth mode of the present invention is, for example, as illustrated in Fig. 3, the image processing apparatus according to the third mode of the present invention in which the opening 42 of the top cover 40 is smaller than a top opening 11 of the bath 10 and positioned inside the top opening 11 of the bath 10 in a plan view, and a region surrounding the opening 42 of the top cover 40 forms a tapered surface inclined downward.

With the configuration as described above, since the region surrounding the opening of the top cover forms the tapered surface, it is easy to insert the test object deep down through this opening, and also it becomes possible to bring the basal portion of the test object closer to the surface of the propagation liquid in the bath, which helps to achieve thorough imaging.

An image processing apparatus according to a fifth mode of the present invention is, for example, as illustrated in Fig. 3, the image processing apparatus according to the third or fourth mode of the present invention in which the top end portion of the bath 10 is positioned lower than the opening 42 of the top cover 40 in height.

With the configuration as described above, when the propagation liquid is supplied into the inside of the bath, the propagation liquid can smoothly flow toward the collection tank before overflowing onto the top cover.

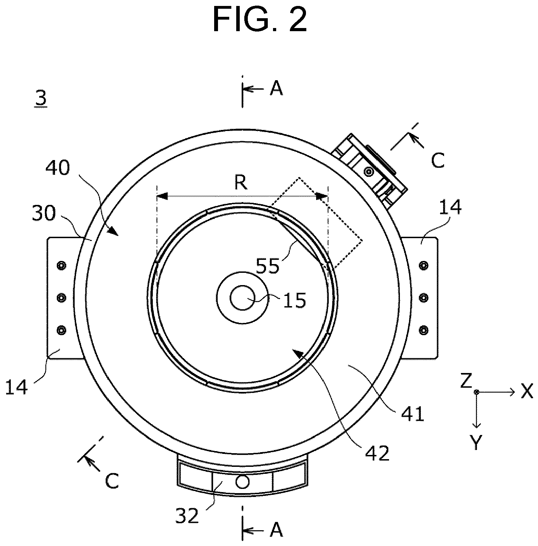

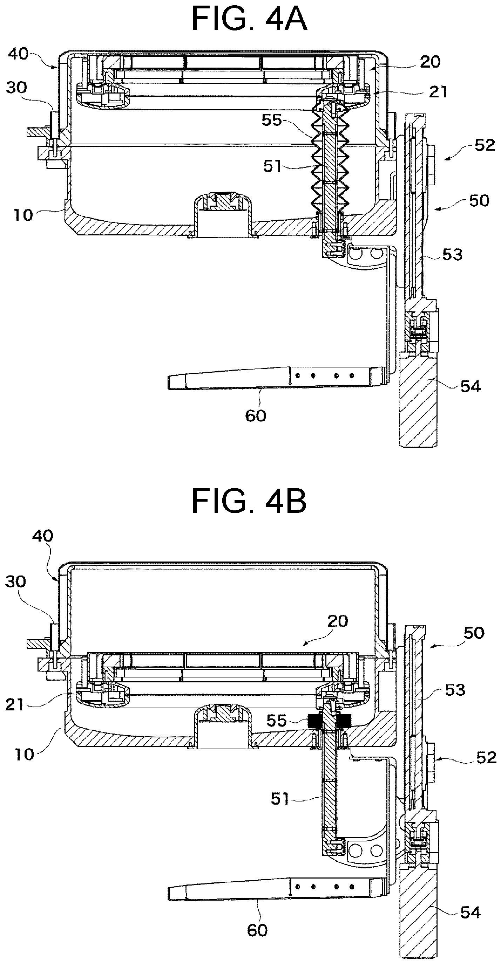

An image processing apparatus according to a sixth mode of the present invention is, for example, as illustrated in Figs. 2 and 4, the image processing apparatus according to any one of the first to fifth modes of the present invention in which the measurement device 20 is formed in an annular shape and disposed in the inside of the bath 10, and a lifter 50 that extends upward from a bottom portion of the bath 10 and moves the measurement device 20 in a height direction is attached to at least one position of a bottom portion of the measurement device formed in the annular shape.

With the configuration as described above, the measurement device in the inside of the bath can be moved in the height direction using a simple configuration, and since the lifter is attached at one position, the dead angle created by the lifter can be minimized in a case where, for example, the inside of the bath is cleaned, and thus it is easy to perform maintenance.

An image processing apparatus according to a seventh mode of the present invention is, for example, as illustrated in Fig. 4, the image processing apparatus according to the sixth mode of the present invention in which an outer periphery of a portion of the lifter 50, the portion being positioned in the inside of the bath 10, is covered by a liquid-tight cover 55 that is extendable in accordance with an operation of the lifter 50.

With the configuration as described above, liquid leakage around the lifter is suppressed by the liquid-tight cover, and a wiring line connected to a transducer can be routed in the liquid-tight cover, and thus there is no need to additionally provide a path for the wiring line of the transducer.

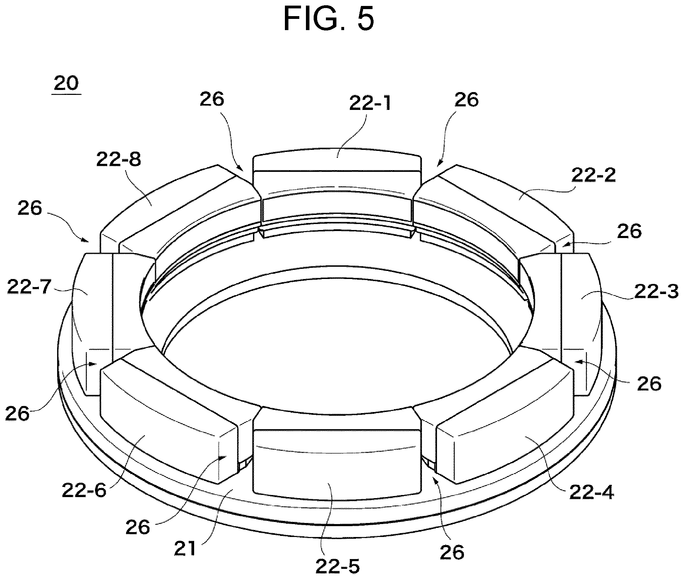

An image processing apparatus according to an eighth mode of the present invention includes, for example, as illustrated in Figs. 5 to 6, the bath 10, the inside of which is filled with the propagation liquid W and in which the test object S is to be immersed; and the measurement device 20, which has the group of elements that irradiates the inside of the bath 10 with the radiation wave and receives the radiation wave, which is a scattered wave, the measurement device 20 including a transducer base 21 formed in an annular shape, and a plurality of transducers 22 disposed at an upper portion of the transducer base 21 such that emission surfaces for the radiation wave face an inner side of the transducer base 21.

With the configuration as described above, the measurement device has the transducer base and the plurality of transducers as separate members and is formed by assembling these members, and thus it is easy to manufacture. In addition, since the measurement device is provided with not a single transducer but a plurality of transducers, the manufacturing yield of a transducer as a unit is high, and when one or some of the transducers fail, only the failed transducer or transducers need to be repaired or replaced, thereby maintainability is increased.

An image processing apparatus according to a ninth mode of the present invention is, for example, as illustrated in Fig. 3, the image processing apparatus according to the eighth mode of the present invention in which the plurality of transducers are each covered by a resin mold 24.

If it is planned to make the plurality of transducers collectively liquid-tight, there may be a problem in that, for example, load is applied to between the transducers, and manufacturing for maintaining a perfect liquid-tight structure is difficult. In contrast, with the configuration as described above, the liquid tightness of each of the plurality of transducers is ensured by the resin mold, and thus it is easy to manufacture. In addition, compared with a case where a plurality of transducers are collectively made liquid-tight by a resin mold, the load from the transducers to the resin molds is suppressed, and a liquid tightness capable of withstanding long-term use can be achieved.

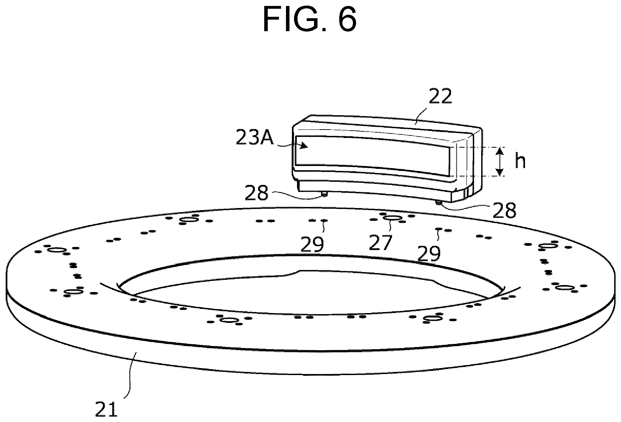

An image processing apparatus according to a tenth mode of the present invention is, for example, as illustrated in Fig. 6, the image processing apparatus according to the eighth or ninth mode of the present invention in which an alignment pin 28 is provided at the plurality of transducers 22 and/or the transducer base 21, and alignment of the plurality of transducers 22 and the transducer base 21 is performed with the alignment pins 28.

With the configuration as described above, the transducers can be assuredly and easily attached to the transducer base by using the alignment pins. Moreover, the transducers themselves are members that generate vibrations, and the transducers can be supported even by the alignment pins, and position shifts of the transducers involved in operation of the transducers can be effectively suppressed.

An image processing apparatus according to an eleventh mode of the present invention is, for example, as illustrated in Fig. 3, the image processing apparatus according to any one of the eighth to tenth modes of the present invention in which a bottom surface of the transducer base 21 has a shape that is convex downward.

With the configuration as described above, air bubbles are less likely to attach to the bottom surface of the vibrating base, and the effect of reflection or the like due to the presence of air bubbles in a radiation field can be eliminated.

An image processing apparatus according to a twelfth mode of the present invention is, for example, as illustrated in Fig. 5, the image processing apparatus according to any one of the eighth to eleventh modes of the present invention in which a gap 26 through which the propagation liquid W is able to pass is formed between the plurality of transducers 22.

With the configuration as described above, even when the transducers, which are separately formed, are operated by vibrations caused at the time of operation, they no longer come into contact with each other. Moreover, since the propagation liquid can flow through this gap, the propagation liquid in the inside of the bath is no longer blocked by the measurement device, and especially when the measurement device is in operation, the disturbance of the flow of the propagation liquid in the inside of the bath can be suppressed.

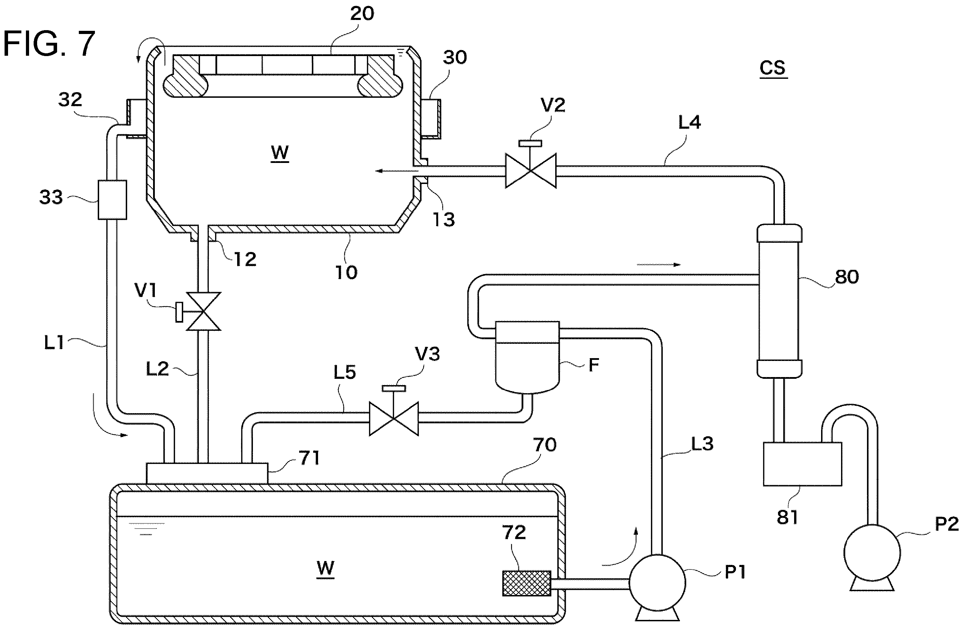

An image processing apparatus according to a thirteenth mode of the present invention is, for example, as illustrated in Fig. 7, the image processing apparatus according to any one of the second to twelfth modes of the present invention which further includes a circulation system CS for circulating the propagation liquid W in the inside of the bath 10, and in which the circulation system CS includes: a storage tank 70 that stores the propagation liquid W; a propagation liquid supply module (V2, P1, L3, L4) for supplying the propagation liquid W in an inside of the storage tank 70 to the bath 10; a collection-tank side drain module (L1) that returns the propagation liquid W collected by the collection tank 30 to the storage tank 70; a bath side drain module (V1, L2) that is provided at the bottom portion of the bath 10 and returns the propagation liquid W in the inside of the bath 10 to the storage tank 70; a filter F that purifies the propagation liquid W to be supplied to the bath 10; and a deaeration module 80 that removes air in the propagation liquid W to be supplied to the bath 10.

With the configuration as described above, the propagation liquid can be repeatedly used because of the circulation system, and the amount of effort needed to prepare the propagation liquid can be reduced. Moreover, the propagation liquid in the circulation system comes from the propagation liquid in the storage tank, and thus the image processing apparatus can be installed at a place where there is not a water source nearby.

An image processing apparatus according to a fourteenth mode of the present invention is, for example, as illustrated in Fig. 7, the image processing apparatus according to the thirteenth mode of the present invention in which the propagation liquid supply module includes a propagation liquid supply valve V2 that controls supply of the propagation liquid, and the propagation liquid supply valve has a supply amount adjustment structure that enables maintaining of supply of a predetermined amount of the propagation liquid while the measurement device is in operation.

With the configuration as described above, even in a case where the liquid level of the propagation liquid in the inside of the bath unintentionally falls while, for example, the measurement device is in operation, the propagation liquid can be promptly added, and the liquid level of the propagation liquid can always be maintained at the highest level.

An image processing apparatus according to a fifteenth mode of the present invention is, for example, as illustrated in Fig. 8, the image processing apparatus according to any one of the first to fourteenth modes of the present invention further including a user interface 150 that allows step-wise setting of the liquid level before the test object S is immersed in the inside of the bath 10, the liquid level being controlled by the liquid level control module 123.

With the configuration as described above, the time required to adjust the liquid level after the test object is immersed in the inside of the bath can be reduced by setting the pre-measurement liquid level through the user interface.

An image processing apparatus control program according to a sixteenth mode of the present invention causes, for example, as illustrated in Figs. 3 and 9 to 10, an image processing apparatus 1 to execute steps described in (1) to (4) below, the image processing apparatus 1 including a bath 10 an inside of which is filled with a propagation liquid W and in which a test object S is to be immersed, a measurement device 20 having a group of elements that irradiates the inside of the bath 10 with a radiation wave and receives the radiation wave, which is a scattered wave, and an upper drainage detection sensor 33 that detects draining of the propagation liquid W from a top portion of the bath 10 to outside the bath 10.
(1) A step for filling the inside of the bath with the propagation liquid an amount of which is smaller than a capacity of the bath by a predetermined amount before the test object is immersed in the inside of the bath;
(2) a step for supplying the propagation liquid into the inside of the bath;
(3) a step for detecting draining of the propagation liquid using the upper drainage detection sensor; and
(4) a step for allowing the measurement device to perform measurement in a case where draining of the propagation liquid is detected.

With the configuration as described above, a pre-measurement liquid level adjustment process can be easily achieved in an image forming apparatus.

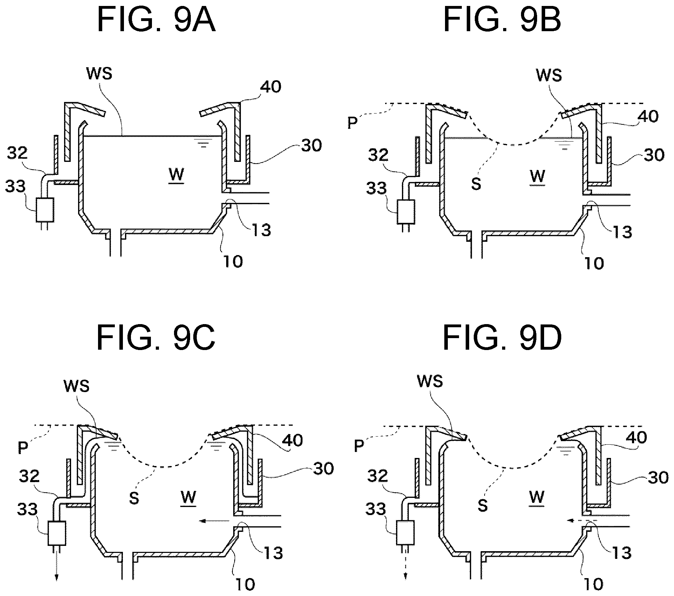

An image processing apparatus control program according a seventeenth mode of the present invention is, for example, as illustrated in Figs. 9 to 10, the image processing apparatus control program according to the sixteenth mode of the present invention further causing the image processing apparatus to execute a step described in (5) below after the step described in the (3).
(5) A step for reducing an amount of the propagation liquid to be supplied into the inside of the bath per unit time or stopping supply of the propagation liquid.

With the configuration as described above, in a case where supply of the propagation liquid is stopped, a liquid stream due to supply of the propagation liquid does not occur in the inside of the bath, and thus the test object is not moved by the liquid stream, and high measurement accuracy can be maintained. Moreover, in a case where supply of the propagation liquid is reduced, even in a case where the propagation liquid is accidentally drained to outside the bath by, for example, movement of the measurement device in the height direction at the time of measurement, the liquid level can be promptly returned back to the original highest level.

An image processing apparatus control program according an eighteenth mode of the present invention is the image processing apparatus control program according to the sixteenth or seventeenth mode of the present invention further causing the image processing apparatus to execute a step described in (6) below in a case where, after the step described in the (4), the test object is moved to outside the bath.
(6) A step for supplying the propagation liquid into the inside of the bath to drain the propagation liquid in the inside of the bath from a top portion of the bath to outside the bath and/or for draining a predetermined amount of the propagation liquid in the inside of the bath from a bottom portion of the bath.

With the configuration as described above, the propagation liquid in the bath is exchanged with purified propagation liquid at a timing at which the test object or the examinee is changed, and thus measurement using a normal propagation liquid is always possible.

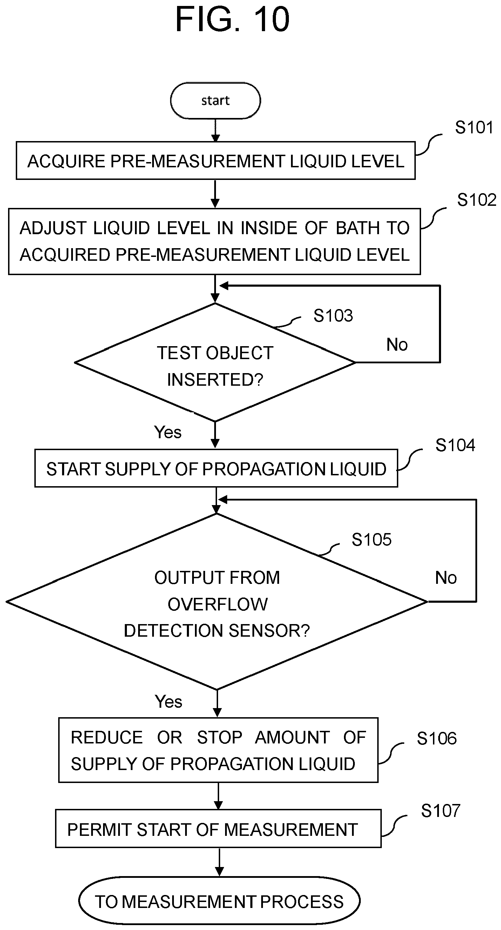

An image processing apparatus control program according a nineteenth mode of the present invention is, for example, as illustrated in Figs. 8 and 10, the image processing apparatus control program according to any one of the sixteenth to eighteenth mode of the present invention in which the predetermined amount in the step described in the (1) is step-wise adjustable.

With the configuration as described above, the time required to adjust the liquid level after the test object is immersed in the inside of the bath can be reduced by step-wise adjusting a pre-measurement liquid level.

### Advantageous Effects of Invention

According to the present invention, in an automatic scanning image processing apparatus of a type in which a test object is immersed in a bath, it becomes possible, with a simple configuration, to immerse the basal portion of the test object in a propagation liquid and to execute thorough imaging (measurement) of the test object.

### BRIEF DESCRIPTION OF DRAWINGS

[Fig. 1] Fig. 1 is an external view of an image processing apparatus according to an embodiment of the present invention.
[Fig. 2] Fig. 2 is a plan view of a measurement apparatus according to the embodiment of the present invention.
[Fig. 3] Fig. 3 includes cross-sectional diagrams of the measurement apparatus according to the embodiment of the present invention, Fig. 3A illustrates a cross section taken along line A-A of Fig. 2, and Fig. 3B illustrates an enlarged cross section of a B portion of Fig. 3A.
[Fig. 4] Fig. 4 includes other cross-sectional diagrams of the measurement apparatus according to the embodiment of the present invention, Fig. 4A illustrates a cross section taken along line C-C of Fig. 2 in a state in which a ring array is at an initial position, and Fig. 4B illustrates a cross section taken along line C-C of Fig. 2 in a state in which the ring array is at an endpoint.
[Fig. 5] Fig. 5 is a perspective view illustrating the ring array according to the embodiment of the present invention.
[Fig. 6] Fig. 6 is a perspective view illustrating a state in which the ring array illustrated in Fig. 5 is partially disassembled.
[Fig. 7] Fig. 7 is a schematic structural diagram schematically illustrating a circulation system for a propagation liquid of the image processing apparatus according to the embodiment of the present invention.
[Fig. 8] Fig. 8 is a functional block diagram of the image processing apparatus according to the embodiment of the present invention.
[Fig. 9] Fig. 9 includes schematic diagrams illustrating changes in liquid level when measurement using the image processing apparatus according to the embodiment of the present invention is started.
[Fig. 10] Fig. 10 is a flowchart illustrating a series of operations when measurement using the image processing apparatus according to the embodiment of the present invention is started.

### DESCRIPTION OF EMBODIMENTS

This application is based on Japanese Patent Application No. 2019-036731 filed in Japan on February 28, 2019, the content of which forms a portion of the content of the present application.

The present invention will be further fully understood from the following detailed description. The range of further applications of the present application will be clear from the following detailed description. However, the detailed description and specific, actual examples are preferred embodiments of the present invention and are described for the purpose of explanation. This is because various changes and modifications are obvious from this detailed description to those skilled in the art within the spirit and scope of the present invention.

The applicant does not intend to dedicate any of the described embodiments to public, and embodiments that may be literally excluded from the scope of the claims out of the disclosed modifications and alternatives are also a portion of the invention under the doctrine of equivalents.

In the following, embodiments of the present invention will be described with reference to the drawings. Note that, in the following, the scope needed to achieve the objective of the present invention will be schematically illustrated, the scope for describing the corresponding portions of the present invention will be mainly described, and the portions for which description is omitted are based on existing technologies.

In the following embodiments of the present invention, a case where an ultrasound diagnostic apparatus is applied as an image processing apparatus, and a breast is applied as a test object will be described in detail. Note that the image processing apparatus and the test object are not limited thereto.

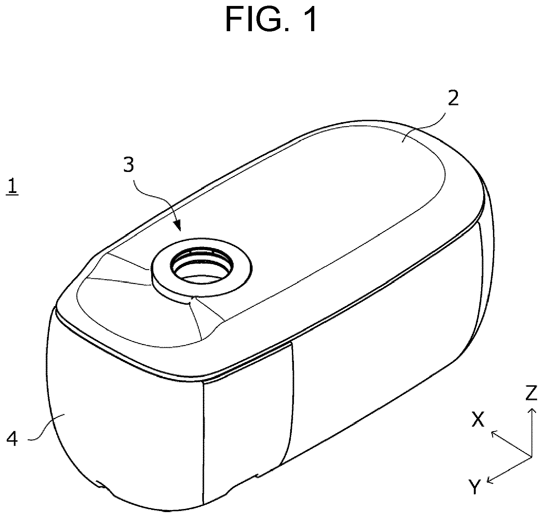

Fig. 1 is an external view of an image processing apparatus 1 according to an embodiment of the present invention. In the drawings according to the embodiments of the present application including this Fig. 1, the width direction (the short side direction) of the image processing apparatus 1 is defined as the X direction, the length direction (the long side direction) thereof as the Y direction, and the up-down direction (the height direction) thereof as the Z direction. The image processing apparatus 1 has an exterior shape formed in a substantially rectangular-parallelepiped shape such that the body of an examinee P (see Fig. 9.) can be placed thereon, and includes a measurement table 2, a measurement apparatus 3, and a cover panel 4.

The measurement table 2 is a table for the examinee P to lie face down on a top portion thereof, and is designed to have a predetermined strength capable of supporting the weight of the examinee P and have a long length in the Y direction of Fig. 1, so that the examinee's head is positioned at a frontward portion in Fig. 1. In particular, for example, the material and shape of the top portion of the measurement table 2 can be adjusted as appropriate by considering, for example, the load on the examinee. The measurement apparatus 3 is provided at a part of the top portion of the measurement table 3 and is a device for measuring a test object S (see Fig. 9.), which is immersed in the inside of the device. A specific configuration and so forth of the measurement apparatus 3 will be described later. Note that the arrangement of the measurement apparatus 3 with respect to the measurement table 2 is adjusted in accordance with the position of a breast (that is, the test object S) of the examinee P lying on the measurement table 2. In the present embodiment, the measurement table 2 and the measurement apparatus 3 are flush with each other such that a gap is not created in a border portion between the measurement table 2 and the measurement apparatus 3; however, the present invention is not limited thereto. Specifically, for example, a predetermined gap may be formed in this border portion and may be used as a water channel to remove a propagation liquid W (see Fig. 7.) in a case where, for example, the propagation liquid W is on the measurement apparatus 3. The cover panel 4 is arranged so as to surround an outer periphery of the measurement table 2 and hides, from for example the examinee, various members and so forth constituting the measurement apparatus 3 disposed inside the measurement table 2. Preferably, the cover panel 4 is configured such that a portion thereof is easily removable, so that it becomes possible, for example, to perform maintenance of the measurement apparatus 3 or to put in and take out a storage tank 70 (see Fig. 7.) for storing the propagation liquid W, which is described later.

Fig. 2 is a plan view of the measurement apparatus 3 according to the embodiment of the present invention, and Fig. 3 includes cross-sectional diagrams of the measurement apparatus according to the embodiment of the present invention. Fig. 3A illustrates a cross section taken along line A-A of Fig. 2, and Fig. 3B illustrates an enlarged cross section of a B portion of Fig. 3A. Note that, in Figs. 2 and 3, illustration of the measurement table 2 and so forth is omitted to facilitate understanding of the configuration of the measurement apparatus 3 itself.

As illustrated in Figs. 2 and 3, the measurement apparatus 3 includes a bath 10 the inside of which is filled with the propagation liquid W and in which the test object S is to be immersed, a ring array (a measurement device) 20 having a group of elements that measures the test object S by irradiating the inside of the bath 10 with radiation waves (ultrasound) (by emitting radiation waves (ultrasound) in the inside of the bath) and receiving scattered radiation waves, a collection tank 30 disposed at or near the outer periphery of the bath 10, and a top cover 40 disposed above the bath 10.

The bath 10 can store the propagation liquid W in the inside thereof, and includes a tub-shaped container with a top opening 11 formed in the top of the container. Moreover, a bottom portion of the bath 10 is provided with a bottom drain hole 12, through which the propagation liquid W stored in the inside of the bath 10 can be drained, and the side of the bath 10 is provided with a liquid feed port 13, through which the propagation liquid W is fed. Preferably, the liquid feed port 13 is provided at a lower position than the bottom surface of the ring array 20, which will be described later, positioned at the top-end position in a measurement process. This is because when the propagation liquid is fed in a state in which the liquid feed port 13 is higher than the bottom surface of the ring array 20, depending on the speed of a liquid stream, a phenomenon may occur in which the propagation liquid is drained to outside the bath 10 along the outside of the ring array 20 while the liquid level in the ring array 20 is not temporarily rising. The bottom portion of the bath 10 is inclined toward the bottom drain hole 12 by about 2 to 3°, and this can prevent the propagation liquid W from remaining at the bottom of the bath 10 at the time of draining. Furthermore, preferably, the capacity of the bath 10 is set to, for example, 16 to 20 liters, which corresponds to the weight of the bath 10 that can be handled by, for example, an operator (regardless of gender) handling the image processing apparatus 1.

Moreover, the side of the bath 10 is provided with installation protrusions 14 extending in a horizontal direction, which are for fixing the bath 10 to the measurement table 2, and the horizontality of the bath 10 is ensured by fixing the installation protrusions 14 horizontally. Here, a horizontal accuracy required for a water surface WS (see Fig. 9.) and the ring array 20 in the bath 10 will be briefly described. In a case where the inside diameter of the ring array 20 is denoted by R (see Fig. 2.), and the height of an emission surface 23A of the ring array 20 is denoted by h (see Fig. 6.), it is desirable that an angle α which the water surface WS forms with the ring array 20 satisfy tanα ≤ h/(5R). This is because if a portion of a group of ultrasonic elements in the ring array 20 is exposed to air, the ultrasound emission-reception sensitivity is reduced by an amount corresponding to the exposed portion, and furthermore ultrasound is not emitted from the exposed portion of the group of ultrasonic elements, and noise resulting from multiple reflection is superimposed on a reception signal. In order to maintain the image quality of image data obtained after measurement, a change in the emission-reception sensitivity needs to be suppressed to 2 dB or less, and a reduction in sensitivity needs to be suppressed to 20% or less. Thus, exposure of the group of ultrasonic elements to air needs to be suppressed to about h/5, and tanα = h/(5R) is an inclination serving as a measure at which exposure starts to affect the image quality.

Moreover, a position detection device 15 used to specify a three-dimensional position of the test object S is provided at substantially the center of the bottom portion of the bath 10. As the position detection device 15, a device obtained by combining, as needed, various devices such as, for example, a camera, an ultrasonic element, and various sensors can be used. Note that Fig. 2 and so forth illustrate cases where the position detection device 15 is disposed at a central portion of the bottom portion of the bath 10 and directed in a vertical direction; however, the position and direction of the position detection device 5 can be adjusted as appropriate in the bath 10.

The ring array 20 is a measurement device for measuring the test object S, is disposed such that the entirety of the ring array 20 is housed in the inside of the bath 10, and operates in a state of being immersed in the propagation liquid W, with which the inside of the bath 10 is filled. The ring array 20 includes a transducer base 21 and a plurality of transducers 22, which are arranged on an upper portion of the transducer base 21. The transducers 22 (eight transducers 22 in the present embodiment) are arranged on the XY place on the transducer base 21 and serve as a group of elements for measuring (imaging) the test object S. The detailed configuration of individual units of the ring array 20 will be described later.

The collection tank 30 is formed to surround the outer side of the bath 10 and is a tank for collecting the propagation liquid W drained (overflowing) from the top opening 11 of the bath 10 to outside the bath 10. A drain hole 31 for draining the propagation liquid W collected by collection tank 30 is formed at a predetermined one position of a lower portion of the side of the collection tank 30, and the bottom surface of the collection tank 30 is inclined toward the side where the drain hole 31 is formed such that the propagation liquid W is guided to the drain hole 31. Moreover, the collection tank 30 includes a collection-tank side drain hole 32, which communicates with the drain hole 31 and is a hole for returning the propagation liquid W collected by the collection tank 30 to the storage tank 70.

The collection-tank side drain hole 32 is provided with an overflow detection sensor (an upper drainage detection sensor) 33, which is a sensor for detecting the flow of fluid flowing through the collection-tank side drain hole 32 and includes, for example, a flow switch, an electromagnetic flow sensor, or the like. The overflow detection sensor 33 can detect draining of the propagation liquid W in the inside of the bath 10 from the top portion of the bath 10 to outside the bath 10 (that is, occurrence of an overflow). Note that the overflow detection sensor 33 in the present embodiment is disposed at the collection-tank side drain hole 32 as described above from the viewpoint of, for example, the ease of installation and the certainty of detection. However, this position is a little far from the bath 10, and thus there is actually a slight time lag between occurrence of an overflow from the bath 10 to detection of the overflow by the overflow detection sensor 33. For example, in cases where, it is desired to reduce, for example, the total time from when the test object S is inserted into the inside of the bath 10 to when measurement of the test object S is started, it is preferable that the overflow detection sensor 33 be arranged at a closer position to the top opening 11 of the bath 10. In this manner, regarding the specific arrangement and type of sensor of the overflow detection sensor 33, changes can be made, as appropriate, as far as the functions described above can be maintained. In the image processing apparatus 1 according to the present embodiment, as will be described in detail in the following, the liquid level in the bath 10 is controlled on the basis of an output from the overflow detection sensor 33. However, it should be particularly noted that the present invention does not intend to exclude installation of another sensor for liquid level detection. That is, for example, an additional liquid level sensor may be provided at a predetermined position in the inside of the bath 10 or at the periphery of the bath 10 for the purpose of complementing liquid level control performed by the overflow detection sensor 33.

The top cover 40 is disposed above the bath 10 and partially covers the top portion of the bath 10. The top cover 40 has a top side 41 and a side wall 43. The top side 41 has a top side opening 42 and is disposed along the XY plane. The top side opening 42 is formed in an annular shape when viewed in a plan view, and the test object S can be inserted through a center portion of the top side opening 42. The side wall 43 is formed so as to extend downward from the outer periphery of the top side 41 in the Z direction by a predetermined distance. As illustrated in Fig. 3, a lower portion of the side wall 43 of the top cover 40 is fixed to the external wall of the bath 10. Note that, preferably, the top cover 40 has a configuration with which at least the top side 41 can be easily removed from the bath 10 (for example, by providing a middle portion of the side wall 43 with a mechanism that enables top-bottom separation). At least the top side 41 of the top cover 40 can be removed from the bath 10, and thus it is easy to clean inside the bath 10. Moreover, in a case where a portion of the test object S is caught between the transducers 22 and the top cover 30, this caught portion can be easily released by lifting the top cover 40 together with the examinee S, thereby improving the level of safety. Furthermore, the top cover 40 is composed of a plurality of members, and thus the level of difficulty of mass-production design can be expected to decrease, and cost reduction can be achieved.

The diameter of the outer periphery of the top side 41 of the top cover 40 is larger than the diameter of the top opening 11 of the bath 10 and smaller than the inside diameter of the collection tank 30. Note that, in the present embodiment, the diameter of the outer periphery of the top side 41 is smaller than the inside diameter of the collection tank 30 but not limited thereto. Specifically, for example, the diameter of the outer periphery of the top side 41 is made equal to the diameter of the collection tank 30, and a lower end portion of the side wall 43 of the top cover 40 may be placed on or connected to an upper end portion of the side wall of the collection tank 30 such that the side wall 43 of the top cover 40 is continuous with the side wall of the collection tank 30. This configuration can prevent, for example, dust from entering the collection tank 30 from outside the measurement apparatus 3. The diameter of the top side opening 42 formed in the top side 41 is smaller than the inside diameter of the top opening 11 of the bath 10 and slightly larger than the inside diameter of the ring array 20.

Furthermore, a region surrounding the top side opening 42 of the top side 41 forms a tapered surface inclined downward by a predetermined angle θ (for example, 1 to 2°) with respect to the XY plane as illustrated in Fig. 3B. The tapered surface formed in this manner helps insertion of the test object S into the top side opening 42, and it becomes easy for the examinee P to perform an operation for inserting the entirety of the test object S into the top side opening 42. In addition, the basal portion of the test object S can be held at a closer position to the bath 10. Specifically, the distance between the top side opening 42 and the ring array 20 supported at the top end position (that is, an initial position illustrated in Figs. 4A and 3A) in the Z direction is 0.5 to 1.5 mm, and the basal portion of the test object S can be positioned close to the bath 10. Note that, since the examinee P lies on the top cover 40 at the time of measurement, preferably, the thickness and materials of the top cover 40 are adjusted as appropriate, so that the top cover 40 does not deform by receiving the weight of the examinee P. Moreover, the outer shape of the top side 41 can be changed as appropriate in accordance with the shape of the bath 10, that of the test object S, or that of the ring array 20. The shape of the top side opening 42 is not necessarily a round shape and can be changed as appropriate to an oval shape, an oblong shape (such that it is easy to also insert a section of his or her armpit portion into the inside of the bath 10), a polygonal shape, or the like.

As illustrated in Fig. 3, the top side 41 of the top side cover 40 covers the entire periphery of the top end portion, which forms the top opening 11, of the bath 10; however, a gap 44 is formed between the top end portion of the bath 10 and the undersurface of the top side 41, which are spaced apart by a predetermined length d in the Z direction. The gap 44 is a gap through which the propagation liquid W overflowing from the top portion of the bath 10 passes. The length (width) d is determined by the top opening 10 included in the top end portion of the bath 10 that is set to be lower than the height of the top side opening 42, and the angle of the tapered surface of the top side cover 40 (which is adjusted so that the examinee P does not experience pain), and needs to be a sufficient width for the propagation liquid W to flow. For example, in a case where the diameter of the top side opening 42 is about 230 mm, and the angle of the tapered surface of the top cover 40 is 1 to 2°, it is preferable that the length d be in the range from 2.0 to 3.0 mm. If the length d is shorter than 2.0 mm, all the overflowing propagation liquid W cannot be guided from the gap 44 to the collection tank 30, and the propagation liquid W flows onto the top cover 40. If the length d is longer than 3.0 mm, the distance between the bath 10 and the top cover 40 is too far, and the propagation liquid W in the bath 10 cannot reach the basal portion of the test object S. Thus, imaging of the basal portion of the test object S cannot be accurately performed. As is clearly understood from the description above, "overflow" in the present embodiment indicates a phenomenon in which the propagation liquid W in the bath 10 flows into the collection tank 30 via the gap 44. For example, a phenomenon in which the propagation liquid W overflows onto the top side 41 of the top cover 40 is not included in "overflow" described here. By forming the gap 44 as described above, when the propagation liquid W is supplied into the inside of the bath 10, the propagation liquid W flows to the collection tank 30 through the gap 44 before the propagation liquid W overflows onto the top cover 40. Thus, by using the gap 44, even for a type of ultrasound diagnostic apparatus in which the test object S is directly immersed in the propagation liquid W in the bath 10, the liquid level of the propagation liquid W in the bath 10 can be easily adjusted to a desired liquid level.

In addition to the configuration described above, a water reception plate 60 is provided below the measurement apparatus 3 as illustrated in Fig. 4. The water reception plate 60 is provided so as not to wet the floor where the image processing apparatus 1 is installed even in a case where the propagation liquid W leaks due to breakage of an accordion portion 55 or from the junction between the accordion portion 55 and the bath 10. The propagation liquid W received by the water reception plate 60 in a case where the leakage described above occurs is guided to a drain strainer 71 of the storage tank 70, which will be described later.

Next, with reference to Figs. 3 to 6, the configuration of the ring array 20 according to the present invention will further be described. Fig. 4 includes other cross-sectional diagrams of the measurement device according to the embodiment of the present invention. Fig. 4A illustrates a cross section taken along line C-C of Fig. 2 in a state in which the ring array is at the initial position, and Fig. 4B illustrates a cross section taken along line C-C of Fig. 2 in a state in which the ring array is at an endpoint. Fig. 5 is a perspective view illustrating the ring array according to the embodiment of the present invention. Fig. 6 is a perspective view illustrating a state in which the ring array illustrated in Fig. 5 is partially disassembled.

Here, regarding a ring array 20 used in a state of being completely immersed in the bath 10 in the same way as the ring array 20 according to the present invention, high waterproofness is desired, and also there are various matters that need to be considered such as the adhesion resistance to air bubbles that may adversely affect imaging and non-disturbance of the flow of the propagation liquid. Regarding the present invention, the structure of the ring array 20 was studied by also considering the viewpoints described above, and the following configuration was conceived. Thus, as another objective of the present invention, an image processing apparatus of the present invention has the objective of providing a ring array (a measurement device) that is appropriate for use in the state of being immersed in a bath.

As described above, the ring array 20 according to the embodiment of the present invention includes the transducer base 21 and the plurality of transducers 22. As illustrated in Figs. 3, 5, and 6, the transducer base 21 is an annular member having a predetermined height and has an annular cavity thereinside such that, for example, wiring lines for the transducers 22 can be routed. The shape of the transducer base 21 may be round, oval, or polygonal. Although the planar shape of the transducer base 21 is described as annular, various shapes are allowed including, for example, a C shaped and a U shape obtained by separating a portion of an annular shape as far as those shapes make it possible to support the ring array 20 horizontally.

In particular, the bottom shape of the transducer base 21 is convex downward, more specifically, an arc shape protruding downward when viewed in cross section. This shape helps to prevent air bubbles from adhering to the bottom portion of the transducer base 21, so that phenomena such as reflection of radiation waves due to air bubbles do not occur, and imaging can be accurately performed. Note that, preferably, not only the bottom surface shape of the transducer base 21 but also a portion thereof being in contact with the propagation liquid W has a shape that is made as smooth as possible in terms of the viewpoint of suppressing adhesion of air bubbles, and thus the entire outer shape may be formed by a smoothly curved plane.

The plurality of transducers (ultrasonic elements) 22 are constituted as one unit by collecting a plurality of (for example, 256) ultrasonic elements mainly having an ultrasound emission function and an ultrasound reception function. The plurality of transducers 22 convert an electric signal into ultrasound (having, for example, a frequency of 2.5 to 3.5 MHz), emit the ultrasound from the emission surfaces 23A (see Fig. 6.) of the plurality of transducers 22, receive scattered waves, which are waves reflected (echoed) from the test object S or waves that have passed through the test object S, convert the received scattered waves into an electric signal, and output the electric signal. In the ring array 20 according to the embodiment of the present invention, eight transducers 22-1 to 22-8 are used as illustrated in Fig. 5. The transducers 22-1 to 22-8 are arranged at equal intervals on the top surface of the transducer base 21 such that the emission surfaces 23A face the inner side direction of the annular transducer base 21 (the center portion of the ring array 20). The size of a circular measurement region defined by the emission surfaces 23A of the plurality of transducers 22 can be a size that allows insertion of the basal portion of the test object S. For example, the diameter of the circular measurement region may be about 200 to 250 mm.

Each of the transducers 22 includes a transducer main body 23, which emits and receives ultrasound as illustrated in Fig. 3. For example, the transducer main body 23 and a wiring line (not illustrated.) for supplying an electric signal and the like to the transducer main body 23 form a unit. The entire unit except for the emission surface 23A, which emits and receives ultrasound for the transducer main body 23, and an opening for taking out the wiring line is covered by a resin mold 24 in a liquid-tight manner. The transducer main body 23 is sealed by the resin mold 24 in a liquid-tight manner. A liquid-tight seal 25 is attached to the opening for taking out the wiring line, and the liquid-tight seal 25 is fit into a wiring line insertion hole 27 formed at an appropriate position of the top surface of the transducer base 21 when the transducer 22 is installed on the top surface of the transducer base 21. An O ring may be used as the liquid-tight seal 25. The wiring line for supplying an electric signal and the like to the transducer main body 23 communicate with the center portion of the liquid-tight seal 25, which couples the transducer 22 to the transducer base 21 in a liquid-tight manner, and this wiring line is routed in the cavity inside the transducer base 21.

In a case where an O ring is used as the liquid-tight seal 25, it is preferable that one wiring line insertion hole 27 be closed with one O ring. In this case, the transducers 22 that are made individually watertight have a flat surface as a result of machining, and it is easy to perform sealing with an O ring. In contrast, in a case where one O ring is arranged so as to straddle the border surface of a plurality of adjacent transducer covers and liquid-tightness is achieved, watertightness is less likely to be maintained at a portion where a step formed at the border surface of a plurality of adjacent transducers 22 is in contact with the O ring. In addition, in a state in which the transducer covers for the plurality of transducers 22 are coupled to each other, in a case where the plurality of transducers 22 and a plurality of wiring line insertion holes 27 are sealed with an O ring on a transducer-by-transducer basis, when a transducer 22 among the transducers 22 is screwed into the base with an O ring interposed therebetween, a parallel force having a different magnitude may be applied to other transducers. As a result, stress is applied to the watertight portions including the border surfaces of the adjacent transducers 22, and the watertightness at the connection portions is less likely to be maintained. Thus, for the purpose of maintaining the watertightness of the transducers 22, it is preferable that the transducers 22 be made individually watertight using a mold and then each of the transducers and the wiring line insertion holes 27 be made individually watertight using the liquid-tight seal 25.

As illustrated in Fig. 6, on the undersurface of each of the transducers 22, there are provided one or more alignment pins 28 for aligning the transducer 22 with respect to the transducer base 21 (two alignment pins for the transducer 22 illustrated in Fig. 6). The transducer 22 and the transducer base 21 are aligned by inserting the alignment pins 28 into pin recesses 29 provided at corresponding positions of the top surface of the transducer base 21. Note that, in the present embodiment, the alignment pins 28 are formed on the transducer 22 side, and the pin recesses 29 are formed on the transducer base 21 side; however, alignment pins may be provided on the transducer base 21 side or both the transducer 22 and the transducer base 21. In the present invention, the arrangement, number, and shape of these are not particularly limited.

The eight transducers 22-1 to 22-8, which are aligned by the alignment pins 28, as illustrated in Fig. 5, are disposed at equal intervals so as to form a gap 26 having a predetermined width between adjacent transducers 22 among the transducers 22. The gap 26 is provided as a water channel for preventing the ring array 20 from hindering the flow of the propagation liquid W in the inside of the bath 10. It is sufficient that the width of the gap 26 be adjusted by considering, for example, properties of the propagation liquid W and the outer shape of the transducers 22. Note that, in particular, when the width of the gap 26 at the height at which the transducer main bodies 23 of the transducers 22 are positioned is too large, since a transducer 22 is not present in portions corresponding to the gaps 26, scattered waves that are waves emitted by the transducers 22 and then reflected by or passed through the test object S cannot be received, so that the measurement accuracy for the test object S decreases. It is thus preferable that the width of the gaps at this portion be adjusted to be as small as possible.

Preferably, the width of the gaps 26 is adjusted to the extent that alignment adjustment is possible. Through this adjustment, as described above, water can flow smoothly around the ring array 20 without lowering the measurement accuracy. For example, in a case where, for example, the ring array 20 is operated to move upward in a state in which the bath 10 is filled with the propagation liquid W up to the top end portion thereof, the propagation liquid can be made less likely to overflow onto the examinee P side from the top side opening 42 of the top cover 40 when the propagation liquid W is pushed up by the top surface of the ring array 20 or when the ring array 20 hinders the flow of functional water to the gap 44. As illustrated in Fig. 5, relatively large gaps may be formed at portions where the transducers 22 are connected to the transducer base 21. This ensures sufficient paths for the propagation liquid W on the inner side of the ring array 20 to flow to outside the ring array 20.

The ring array 20 is movable in the inside of the bath 10 in the Z direction using a lifter 50 as illustrated in Fig. 4. Information on tissues of the entirety of the inside of the test object S can be obtained by performing ultrasound measurement while moving the ring array 20 using the lifter 50, and repeatedly acquiring a captured image of a cross section of the test object S while changing the position of the cross section. Scan intervals are, for example, 1.0 mm. Thus, in a case where the depth of a measurement range is 20 mm, 21 tomographic images are to be acquired in total. The scan intervals are not limited to 1.0 mm and can be changed as appropriate to, for example, 0.5 mm or 2.0 mm. Moreover, the depth of the measurement range can be adjusted as appropriate in accordance with the size of the test object S as far as the depth of the measurement range is less than or equal to the length of the Z-direction stroke of the ring array 20 (for example, 100 mm). Note that the scan direction may be from top to bottom or from bottom to top; however, it is preferable that scanning be performed in one direction. In the present embodiment, a state in which the ring array 20 is positioned at an upper position of the bath 10 (the state illustrated in Figs. 4A and 3A) is treated as an initial position for measurement, and a state in which the ring array 20 is positioned at a lower position of the bath 10 (the state illustrated in Fig. 4B) is treated as an endpoint for measurement.

The lifter 50 is attached to one position at the bottom portion of the transducer base 21 and operates the ring array 20 in the Z direction. The lifter 50 has an operative mechanism using a ball screw and includes a support rod 51, an operation unit 52, a screw shaft 53, and a motor 54. The support rod 51 includes a long bar-like member, an end thereof is fixed to the bottom portion of the transducer base 21, the support rod 51 passes through the bottom surface of the bath 10 in a liquid-tight and slidable manner, and the other end thereof is attached to the operation unit 52. One end of the operation unit 52 is attached to the support rod 51, and the other end is screwed together with the screw shaft 53 to form a nut. The operation unit 52 linearly moves in the Z direction in accordance with rotation movement of the screw shaft 53. One end of the screw shaft 53 is coupled to the motor 54, and the screw shaft 53 moves the operation unit 52 by rotating. The motor 54 is a drive source for rotating the screw shaft 53. Note that, in the present embodiment, the operative mechanism using a ball screw is used as the operative mechanism of the lifter 50; however, a freely chosen operative mechanism that can perform linear movement (for example, a linear motor, an electric slider, an electric cylinder, or the like) can be used.

At or near the outer periphery of the support rod 51 positioned between the portion of the transducer base 21 to which the support rod 51 is fixed and the portion of the bottom portion of the bath 10 through which the support rod 51 passes, the accordion portion (a liquid-tight cover) 55 is provided so as to cover the support rod 51. The accordion portion 55 includes a member that is expandable and retractable in the Z direction so as to follow operation of the support rod 51, and is a liquid-tight cover provided to achieve an objective of, for example, preventing the propagation liquid W from entering the portion of the bottom portion of the bath 10 through which the support rod 51 passes. Inside the accordion portion 55 (or the support rod 51), the wiring lines routed in the cavity inside the transducer base 21 for the individual transducers 22 are routed in a collective manner and connected to, for example, an external power source.

As illustrated in Figs. 2 and 4, the accordion portion 55 and the support rod 51 covered by the accordion portion 55 are disposed only at one position of the bottom portion of the transducer base 21. With this configuration, in a case where, for example, the inside of the bath 10 is to be cleaned, a blind spot due to the support rod 51 and the accordion portion 55 can be minimized. Compared with a case where a structure is used in which the transducer base 21 is supported at a plurality of positions, the maintainability is higher. Note that since the transducer base 21 is fixed only at one position by the support rod 51, the transducer base 21 is supported by the support rod 51 in a cantilever state. Thus, it should be particularly noted that in order to reduce the possibility that the transducer base 21 cannot stay horizontal due to the moment with respect to the point to which the support rod 51 is fixed, materials of individual members and a fixing structure between the transducer base 21 and the support rod 51 need to be selected as appropriate. Specifically, as described above, the fixing structure and so forth need to be selected such that the state satisfying tanα ≤ h/(5R) can be maintained for the angle α, which is the angle formed by the water surface WS and the ring array 20. Note that it is sufficient that the support rod 51 and so forth be arranged at at least one position. The position of the support rod 51 and so forth is not limited to the above-described one position, and the support rod 51 and so forth may be disposed at a plurality of positions.

Next, a circulation system CS of the image processing apparatus according to the embodiment of the present invention will be described in the following with reference to Fig. 7. Fig. 7 is a schematic structural diagram schematically illustrating the circulation system CS for the propagation liquid W of the image processing apparatus 1 according to the embodiment of the present invention. The circulation system CS includes the storage tank 70, a collection-tank side drain module, a bath side drain module, a propagation liquid supply module, a hollow fiber filter (a filter) F, and a deaeration module 80. The storage tank 70 stores the propagation liquid W. The collection-tank side drain module returns the propagation liquid W collected by the collection tank 30 to the storage tank 70. The bath side drain module is provided at the bottom portion of the bath 10 and returns the propagation liquid W in the inside of the bath 10 to the storage tank 70. The propagation liquid supply module is a module for supplying the propagation liquid W in the inside of the storage tank 70 to the bath 10. The hollow fiber filter F purifies the propagation liquid to be supplied to the bath 10. The deaeration module 80 removes air inside the propagation liquid W to be supplied to the bath 10.

The storage tank 70 has a sufficient capacity for circulation in the circulation system CS, which is, for example, a capacity of about 20 liters. The propagation liquid W is stored in the inside of the storage tank 70, and for example tap water or the like can be used as the propagation liquid W. The top portion of the storage tank 70 is provided with the drain strainer 71 serving as a drain hole that receives the propagation liquid W drained from the bath 10 and the like, and the side of the storage tank 70 is provided with a water supply strainer 72 serving as a liquid supply port for supplying the propagation liquid W into the inside of the bath 10. Preferably, the storage tank 70 is disposed on a base on casters, which is not illustrated, in order to facilitate changing of the propagation liquid W inside the storage tank 70.

The collection-tank side drain module is a module for returning the propagation liquid W collected by the collection tank 30 to the storage tank 70, for example, when measurement of the test object S is started, and includes a collection-tank side drain pipe L1. The collection-tank side drain pipe L1 is a pipe that couples the collection-tank side drain hole 32 to the drain strainer 71, and the collection side drain pipe L1 is always open.

The bath side drain module is used when the liquid level of the propagation liquid W is adjusted, the inside of the bath 10 is cleaned, or the propagation liquid W is changed, and is a module for returning the propagation liquid W in the inside of the bath 10 to the storage tank 70. The bath side drain module includes a bath side drain pipe L2 and a drain valve V1. The bath side drain pipe L2 is a pipe that couples the bottom drain hole 12 to the drain strainer 71, and includes, for example, a pipe having a larger diameter than the collection side drain pipe L1. The drain valve V1 is disposed in the bath side drain pipe L2, and controls draining of the propagation liquid W in the inside of the bath 10.

The propagation liquid supply module is a module for supplying the propagation liquid W in the inside of the storage tank 70 to the bath 10, and includes a circulation pump P1, supply pipes L3 and L4, and a propagation liquid supply valve V2. The circulation pump P1 and the supply pipes L3 and L4 are items for sending the propagation liquid W in the inside of the storage tank 70 to the bath 10. The propagation liquid supply valve V2 is a valve for controlling the amount of the propagation liquid to be supplied into the inside of the bath 10 among the propagation liquid W sent by the circulation pump P1 and so forth. As the propagation liquid supply valve V2, a valve that can perform supply amount adjustment, that is, valve opening degree adjustment can be used such as a butterfly valve, a ball valve, a needle valve, or the like. In the present embodiment, as the propagation liquid supply valve V2, a butterfly valve is used because, for example, the amount of leakage does not necessarily need to be zero as will be described later.

The hollow fiber filter F is disposed in the supply pipe L3, and purifies the propagation liquid W flowing through the supply pipe L3. The image processing apparatus 1 according to the present embodiment is a type of apparatus that performs measurement on the test object S, which is directly inserted into and immersed in the inside of the bath 10, and thus sebum and so forth in the propagation liquid W are removed by the hollow fiber filter F. As the hollow fiber filter F according to the present embodiment, a filter with a cleaning function is used. The hollow fiber filter F further includes a filter cleaning pipe L5 and a filter cleaning valve V3 for allowing the propagation liquid W to pass at the time of cleaning (backwash). As the hollow fiber filter F with a cleaning function, an existing and known filter can be used, and a description of a detailed configuration and so forth is omitted here. Note that, the hollow fiber filter F does not necessarily need to have the cleaning function described above, and for example a known filter that is periodically replaced and used can also be used.

The deaeration module 80 removes gas (air bubbles) inside the propagation liquid W supplied from the supply pipe L3. For example, a deaeration module having a tubular hollow fiber module therein can be used. The deaeration module 40 further includes a vacuum pump P2 for drawing gas (air bubbles) inside the propagation liquid W, and a trap 81 for capturing liquid (the propagation liquid W) that may flow into the vacuum pump P2 side together with gas. Note that an existing, known module can be used also for the deaeration module 80, and thus a description of a detailed configuration and so forth is omitted here. Moreover, regarding the deaeration module 80, the structure thereof is not limited to the above-described structure using the vacuum pump P2 and so forth. For example, a temperature control mechanism can substitute for the function of the deaeration module 80. That is, the temperature of the functional water W is increased to increase the saturated vapor pressure and reduce the amount of gas dissolved in water, and as a result, deaeration of the functional water W is also possible.

In the circulation system CS having the above-described series of configurations, a circulation path of the propagation liquid W indicated by arrows in Fig. 7 is formed. That is, for example, when the image processing apparatus 1 starts measurement, the propagation liquid W in the inside of the storage tank 70 is subjected to purification processing and deaeration-defoaming processing by the hollow fiber filter F and the deaeration module 80, and then is supplied from the liquid feed port 13 into the inside of the bath 10. Thereafter, the propagation liquid W overflows from the top opening 11 of the bath 10, and is returned into the inside of the storage tank 70 via the collection tank 30 and the collection-tank side drain pipe L1. By using the circulation system CS described above, the image processing apparatus 1 can be installed even at a place there is not a water supply source nearby. Moreover, the propagation liquid W to be supplied to the bath 10 is always subjected to purification processing and deaeration processing by the hollow fiber filter F and the deaeration module 80, and thus the state of the propagation liquid W supplied to the bath 10 can be maintained for a long period, and thus the replacement frequency of the propagation liquid W in the storage tank 70 can be reduced (for example, about once a day). Furthermore, since the hollow fiber filter F and the deaeration module 80 are provided, purified deaerated water or the like does not need to be additionally prepared, and low-priced tap water can be used as water in the storage tank 70 (the propagation liquid W), and therefore measurement of the test object S can be achieved at low cost. As a matter of course, in the circulation system CS according to the present embodiment, various configurations other than the above-described configuration can be used. For example, a heater for increasing the temperature of the propagation liquid W to about temperatures at which the examinee P is not uncomfortable, a disinfection apparatus for disinfecting the propagation liquid (using, for example, ultraviolet rays, ozone water, or chemicals), or a water pressure sensor for detecting the amount of supply or drainage of the propagation liquid W to or from the bath 10 can be arranged at an appropriate position in the circulation system CS.

Next, with reference to Fig. 8, a control module of the image processing apparatus according to the embodiment of the present invention will be described. Fig. 8 is a functional block diagram of the image processing apparatus according to the embodiment of the present invention. Note that Fig. 8 mainly illustrates modules especially associated with the present invention among various modules that the image processing apparatus includes, and thus it goes without saying that the image processing apparatus may include various modules other than the ones illustrated in Fig. 8.

In the image processing apparatus 1 according to the present embodiment, a control box 100 for controlling the entirety of the image processing apparatus 1 is provided. The control box 100 includes an image control unit 110, a mechanical control unit 120, a database 130, and a personal computer (PC) 140.

The image control unit 110 is a unit for performing various types of control to measure the test object S and acquire image data. The image control unit 110 includes at least a signal acquisition module 111, a signal processing module 112, and a position detection module 113. The signal acquisition module 111 is connected to wiring lines of the plurality of transducers 22 of the ring array 20, and is a module for acquiring ultrasonic signal data obtained by performing reception at the transducers 22 and conversion into electric signals. The signal processing module 112 is a module for reconstructing the ultrasonic signals acquired by the signal acquisition module 111 and generating RAW data of a B mode image. The position detection module 113 is a module for detecting position information regarding the test object S detected by the position detection device 15 and for detecting the position of the test object S on the XY plane and a depth position of an imaging region of the test object S in the Z direction.

The mechanical control unit 120 performs mechanical control on various configurations that the image forming apparatus 1 has. The mechanical control unit 120 includes at least a transducer control module 121, a lifter control module 122, and a liquid level control module 123. The transducer control module 121 is a module for operating the plurality of transducers 22 of the ring array 20, and for causing the transducers 22 to output ultrasonic signal data for generating a B mode image of the test object S by operating the transducers 22 at predetermined timings. The lifter control module 122 is a module for operating the support rod 51 in the Z direction through operation of the motor 54 to control the depth direction position of the ring array 20. The liquid level control module 123 is connected to various members in the circulation system CS, and is a module for executing supply and drainage of the propagation liquid W into and from the inside of the bath 10 to keep the liquid level of the propagation liquid W in the inside of the bath 10 at a desired liquid level. The liquid level control module 123 achieves liquid level control of the bath 10 by controlling the valves V1 to V3 and the pumps P1 to P2 on the basis of, for example, outputs from the overflow detection sensor 33 and the position detection device 15.

The database 130 stores a control program for controlling individual modules in the image control unit 110 and the mechanical control unit 120, and includes a known storage medium such as, for example, a hard disk drive (HDD) or a solid-state drive (SSD). The control program is stored, for example, in a software format in the database 130. A series of measurement processes for the test object S is achieved by the CPU of the PC 140, which will be described later, or possibly each module in the image control unit 110 and the mechanical control unit 120 directly executing this software.

The PC 140 is a known personal computer including, for example, a CPU and a memory. The PC 140 controls various types of module by referring to the control program stored in the database 130 and executes various types of data processing on the RAW data generated by the image control unit 110, on the basis of the control program stored in the database 130. The PC 140 is connected to the image control unit 110, the mechanical control unit 120, and the database 130 via an internal bus of the control box 100.

The PC 140 is also connected to a user interface 150 disposed outside the control box 100 and by extension outside the measurement table 2. In the present embodiment, the user interface 150 is provided in a graphical user interface (GUI) format and includes a display module 151 including a liquid crystal monitor or an organic electroluminescent monitor, a keyboard, a pointing device including a mouse or a track ball, and/or an operation module 152 including a touch panel. As the user interface 150, a known terminal device such as a tablet terminal or a notebook personal computer can be used. By using the user interface 150 described above, an instruction from the operator can be acquired at the time of image measurement. Regarding the user interface 150, various changes are allowed such as use of a known voice input-output module as needed or use of a read module including a barcode reader for inputting, for example, examinee information or operator information.

Data of a plurality of slice images of the test object S obtained by performing data processing in the PC 140 is transmitted to a medical image management system (Picture Archiving and Communication System (PACS)) 160 via a network NW including the Internet or an intranet. The transmitted data of slice images is adjusted to have a format based on the Digital Imaging and Communication in Medicine (DICOM) standard so as to be stored in the PACS 160.

Next, a measurement process in the image processing apparatus 1 according to the present embodiment will be briefly described. After various pre-measurement adjustments are complete such as insertion of the test object S into the inside of the bath 10, alignment of the test object S, or movement of the ring array 20 to the initial position for measurement, when starting of measurement is triggered by, for example, a command from the operator, while executing scanning with the transducers 22 in coordination with movement of the ring array 20, the transducer control module 121 and the lifter control module 122 transmit pieces of ultrasonic signal data obtained through reception and conversion performed at the transducers 22 to the signal acquisition module 111 in succession. When scanning of the entire imaging range is complete, a plurality of pieces of ultrasonic signal data obtained through a series of measurement operations are reconstructed into a series of pieces of RAW data by the signal processing module 112. The reconstructed pieces of RAW data are converted by the PC into slice image data based on the DICOM standard, and the slice image data is transmitted via the network NW to and stored in the PACS 160. The series of operations described above is executed on a test object S basis. The stored slice images are associated with, for example, an electronic medical chart of the target test object and are used when a doctor makes a diagnosis or the like. Note that the measurement process for the image processing apparatus 1 according to the embodiment of the present invention is not limited to particular processes. For example, a measurement process that has been hitherto executed in automatic scanning image processing apparatuses as described in, for example, PTLs 1 and 2 may also be used. Thus, a description of details of the measurement process will be omitted.

Next, with reference to Figs. 9 and 10, a liquid level adjustment process related to a main configuration of the image processing apparatus according to the embodiment of the present invention and performed when measurement is started (and before the measurement process is started) will be described in the following. Fig. 9 includes schematic diagrams illustrating changes in liquid level when measurement using the image processing apparatus according to the embodiment of the present invention is started. Fig. 10 is a flowchart illustrating a series of operations performed when measurement using the image processing apparatus according to the embodiment of the present invention is started. Note that members such as the ring array 20 are omitted in Fig. 9 to facilitate observation of the flow of the propagation liquid W.

In the image processing apparatus 1 according to the present embodiment, a notebook personal computer is arranged as the user interface 150 near the measurement table 2. While operating this notebook personal computer, the operator (a laboratory technician, a doctor, or another medical worker) instructs the examinee P to, for example, maintain a predetermined posture or change the test object. Note that the usage pattern of the present invention is not limited to this, and various forms may be used. For example, all instructions to the examinee P are provided by automatic voice or displayed, and the series of operations is automatically performed by the image processing apparatus 1 on the basis of information input by, for example, the examinee P. Prior to measurement, for example, the examinee P is identified, and information on the examinee P is input. These processes may be ones generally performed when measurement is performed using various modalities, and thus a description of details thereof is omitted.

In a case where measurement processing is to be executed using the image processing apparatus 1 on a test object S of a specific examinee P, before the test object S is inserted into the inside of the bath 10, the liquid level inside the bath 10 at this point in time (referred to as pre-measurement liquid level) is determined. Preferably, the pre-measurement liquid level is a liquid level lower than the top end position of the bath 10, and more specifically, the liquid level in the bath 10 is positioned at around a position slightly below the highest liquid level of the bath 10 when the test object S is inserted into the bath 10. This is preferable in terms of prevention of the propagation liquid W from overflowing onto the top side of the top cover 40 when the test object S is inserted and reduction of the time required for a propagation liquid supply process after the test object S is inserted. Preferably, the pre-measurement liquid level is determined by an operation input through the user interface 150 by the operator. In this case, the operator determines or estimates the volume of the test object S by referring to the electronic medical chart acquired in advance or by performing a visual check or a manual examination, and inputs a matching pre-measurement liquid level. When this input is performed, as an input method, a method is preferred in which step-wise inputting is allowed. For example, the operator selects one out of three levels of "large", "medium", and "small" as the size of the test object S to perform an input operation. Note that instead of the operator inputting an input, the PC 140 may automatically input the pre-measurement liquid level by recognizing information on, for example, the electronic medical chart including various types of information on the examinee P.

When the operator inputs the pre-measurement liquid level, the PC 140 acquires the input pre-measurement liquid level (S101), and the liquid level control module 123 executes liquid level control so as to adjust the liquid level in the bath 10 to this pre-measurement liquid level (S102). This liquid level control is achieved mainly by operating the drain valve V1, the propagation liquid supply valve V2, and the circulation pump P1. In this case, a sensor for calculating the amount of supply or drainage of the propagation liquid W or a known sensor can be used in a specific method for detecting the liquid level in the bath 10; however, a high-accuracy sensor or the like is not necessarily needed because a high degree of accuracy is not needed for detection of the pre-measurement liquid level.

When the liquid level in the bath 10 is adjusted to the pre-measurement liquid level by the liquid level control module 123 (the state in Fig. 9A), the examinee P inserts the test object S into the inside of the bath 10 as instructed by the operator. When the test object S is normally inserted (Yes in S103, the state in Fig. 9B), the subsequent propagation liquid W supply process (S104) is executed. When the test object S is not normally inserted (No in S103), the process waits until the test object S is normally inserted. In this case, the "normally" inserted test object S indicates that, for example, a shift of the test object S on the XY plane with respect to the center line of the ring array 20 is within a preset allowable range and that the level of insertion of the test object S is greater than or equal to a predetermined threshold in the Z direction. To detect this insertion state, the position detection device 15 and the position detection module 113 are used.

When the test object S is normally inserted (Yes in S103), the liquid level control module 123 starts supplying the propagation liquid W (S104) Supply of the propagation liquid W is executed when the circulation pump P1 is operated and the propagation liquid supply valve V2 is opened. Faster supply speeds are desirable for the propagation liquid W because an increase in liquid level can be achieved in a short time. However, if the supply speed is too fast, a liquid stream, which may cause noise at the time of measurement, occurs in the bath 10, and time to wait for this liquid stream to settle down is additionally needed. Furthermore, the speed at which the propagation liquid W enters the gap between the test object S and the top side 41 becomes faster than the speed at which the propagation liquid W is collected by the collection tank 30 via the gap 44, and thus the propagation liquid W may overflow onto the top side 41. Therefore, preferably, the supply speed is set to, for example, about 1.6 to 2.0 l/min.

After a predetermined period of time has elapsed after the start of supply of the propagation liquid W, the inside of the bath 10 is filled with the propagation liquid W, and an overflow occurs from the top opening 11 of the bath 10 (see Fig. 9C). The propagation liquid W that has overflowed from the top opening 11 flows into the collection tank 30 through the gap 44 between the bath and the top cover 40, and thereafter flows to the collection-tank side drain hole 32. The overflow detection sensor 33 provided at the collection-tank side drain hole 32 detects the propagation liquid W flowing into the collection side drain hole 32 (Yes in S105). In this case, as illustrated in Fig. 9C, due to the gap 44, which is relatively narrow, and the above-described tapered surface formed around the opening 42 of the top cover 40, a portion of the test object below the top opening 42 is positioned lower than the water surface WS of the propagation liquid W and is in a state of being immersed in the propagation liquid W. As a result, the propagation liquid W reaches even the region between the top opening 42 and the basal portion of the test object S.

Upon detecting an output from the overflow detection sensor 33, the liquid level control module 123 operates the propagation liquid supply valve V2 to substantially reduce or stop the amount of the propagation liquid W to be supplied into the inside of the bath 10 (S106). Here, substantial reduction of the amount of the propagation liquid W to be supplied can be achieved, for example, by using a butterfly valve allowing a predetermined amount of leakage as the propagation liquid supply valve V2 or by providing the supply pipe L4 with a bypass path bypassing the propagation liquid supply valve V2 and having a diameter sufficiently smaller than the diameter of the supply pipe L4. In addition, the substantial reduction of the amount of the propagation liquid W to be supplied indicates that supply of a small amount of the propagation liquid is maintained also in the subsequent measurement process. As a result of this, the image processing apparatus 1 has an advantageous effect in that in a case where the water surface WS that has once reached the top end liquid level, that is, the highest level of the bath 10 has moved downward as a result of reduction of the propagation liquid W due to various factors, the water surface WS that has moved downward can be promptly returned to the highest level since supply of a small amount of the propagation liquid W is maintained. This point is particularly preferable because the image processing apparatus 1 has the lifter 50 and the accordion portion 55, and the liquid level can be promptly returned to the highest level even in a case where when these increase in length in the inside of the bath 10 (that is, the ring array 20 is moved upward), the propagation liquid W overflows to the collection tank 30 side due to an increase in the volume of the lifter 50 and the accordion portion 55.

In addition, stopping supply of the propagation liquid W has an advantageous effect in terms of noise suppression at the time of measurement because a flow due to the propagation liquid W does not occur in the bath 10. Note that, in this case, it can be expected that the liquid level slightly decreases as, for example, the ring array 20 operates; however, a decrease in the liquid level described above does not become a major problem, for example, in a case where the ring array 20 has completed measurement of the basal portion of the test object S, and thus supply of the propagation liquid W can be stopped in such a case. Thus, by considering various conditions, the operator or the like can set settings as to the way in which the propagation liquid W is to be supplied after an output is obtained from the overflow detection sensor 33. As a matter of course, the amount of the propagation liquid to be supplied after an output is obtained from the overflow detection sensor 33 is not necessarily controlled in one mode, and control may be performed by performing switching (between reduction and stopping of the amount of the propagation liquid to be supplied) as appropriate in accordance with, for example, the situation inside the bath 10.

Fig. 9D illustrates the state of the bath 10 after the amount of the propagation liquid to be supplied is reduced in S106. As is clear from Fig. 9D, the water surface WS of the propagation liquid W is in a state of being bowed upward from the height of the top opening 11 due to the action of the surface tension. Thus, the water surface WS reaches the height of the top side opening 42. As a result of this, the test object S positioned below the top side opening 42 is in the state of being immersed in the propagation liquid W. In addition, the propagation liquid W reaches the region between the top opening 42 and the basal portion of the test object S at this point in time, and the propagation liquid W stays so as to fill the gap between the top opening 42 and the basal portion of the test object S also due to the action of the surface tension. As a result, the test object S inserted through the top side opening 42 is in a state in which the test object up to its basal portion is thoroughly immersed in the propagation liquid W, thereby enabling measurement of the entirety of the test object S.

When supply of the propagation liquid W is reduced or stopped, the PC 140 determines that the water surface WS of the propagation liquid W is at the highest liquid level and allows measurement (S107). Note that, even in this case, the measurement process cannot be started as a matter of course if conditions other than the liquid level are not met. For example, in a case where as a result of detecting the position of the test object S in the Z direction by operating the position detection device 15 and the position detection module 113 again after S106, it is detected that for example the basal portion of the test object S is not completely inserted into the top opening 42, the test object S needs to be moved, and the liquid level needs to be readjusted. That is, in S107, it is only determined whether the measurement process can be started from the viewpoint of liquid level adjustment.

As described above, in a type of automatic scanning image processing apparatus in which a test object is immersed in the inside of a bath, the present invention can cause a basal portion of the test object to be immersed in a propagation liquid with an extremely simple configuration that causes the propagation liquid to overflow from the bath and detects only the overflow. Thus, imaging (measurement) of the test object can be thoroughly executed without using a complex configuration such as various sensors.

Optionally, it is preferable that the image processing apparatus 1 according to the embodiment of the present invention have the function of cleaning the propagation liquid W in the bath 10, for example, at a timing at which the examinee P is changed. Specifically, for example, when measurement of the test object S is complete, and the examinee P gets off from the measurement table 2, it is recommended that the propagation liquid W in the bath 10 be cleaned in response to detection of this action or an input operation performed by the operator. It is recommended that this cleaning operation be executed mainly by selecting one out of or combining two methods below.

A first cleaning operation is to supply a predetermined amount of the propagation liquid (preferably, an amount sufficiently greater than the amount of the propagation liquid supplied at the time of liquid level control) from the liquid feed port 13 by operating the propagation liquid supply module, to intentionally cause the propagation liquid W of the top portion of the bath 10, in which the test object S was immersed, to overflow, and to return the propagation liquid W to the storage tank 70 together with contamination such as sebum floating in the bath 10. A second cleaning operation is to open the drain valve V1 in order to eliminate dirt and the like deposited and stayed on the bottom of the bath 10, and to return a predetermined amount of the propagation liquid W including the dirt and the like on the bottom of the bath 10 to the storage tank 70.

By having the cleaning function as described above, the bath 10 can always store a clean propagation liquid W every time the examinee P is changed, and thus the examinee P can undergo measurement without worrying about sanitary conditions.

The transducers 22 are made liquid-tight by a resin mold in the embodiment described above; however, by considering the level of water resistance of the mold material, it is optionally preferable that the transducers 22 be regularly removed to outside the propagation liquid W. Thus, when the examinee P is changed or after the above-described cleaning operation, it is recommended that the liquid level of the propagation liquid W be made lower than or equal to the height of the ring array 20.

### (Other Embodiments)

The image processing apparatus 1 according to the embodiment described above is configured such that the gap 44 is formed between the top end portion of the bath 10 and the undersurface of the top side 41, and when the propagation liquid W in the inside of the bath 10 overflows, the propagation liquid is drained from the top portion of the bath 10 to outside the bath 10 via the gap 44; however, the structure that drains the propagation liquid W to outside the bath 10 is not limited to the above-described configuration. For example, it is sufficient that the position from which the propagation liquid W is drained to outside the bath 10 be at an upper portion of the bath 10, and the image processing apparatus does not necessarily have a structure that causes the propagation liquid W to overflow from the top end portion of the bath 10 as in the above-described embodiment. Thus, as another embodiment of the present application, it is possible to use a structure in which a plurality of drain holes are formed at upper positions of the side wall of the bath 10 and that drains the propagation liquid W in the bath 10 to outside the bath 10 via the drain holes. In a case where such an embodiment is used, the top end portion of the bath 10 can be configured to support directly or indirectly the area around the test object S, and as a result, the top cover 40 can be omitted. Note that the drain holes need to be disposed at height positions higher than or equal to the height of the top end of the ring array 20 arranged at the top end position (initial position) in the measurement process such that the entirety of the ring array 20 is always immersed in the propagation liquid W at the time of measurement.

Note that the image processing apparatus 1 of the present invention achieves a series of control processes by executing the control program stored in a software format in the database 130; however, instead of this, the series of control processes can also be achieved using an application specific integrated circuit (ASIC), a field programmable gate array (FPGA), another complex programmable logic device (CPLD), or a simple programmable logic device (SPLD) in which a dedicated hardware circuit is built.

The present invention is not limited to the above-described embodiments and can be executed by adding various changes thereto without departing from the gist of the present invention. They are all included in the spirit of the present invention.

For all of the documents including the publications, patent applications, and patents cited herein, each document is individually specifically illustrated and is incorporated herein by reference to the same extent that the entire content thereof is described here.

Use of nouns and similar demonstratives used in association with the description of the present invention (especially, in association with the claims below) is construed to cover both singular nouns and plural nouns unless otherwise indicated herein or clearly contradicted with the context. Words such as "provided with", "have", "include", and "incorporate" are construed as open-end terms (that is, meaning "include ... but not limited to ...") unless otherwise noted. Unless otherwise specified herein, details of numerical ranges are only intended to simply serve as an abbreviated form for referring to each of the values within the ranges, and each value is incorporated herein as if the values are individually listed herein. Unless otherwise indicated herein or clearly contradicted with the context, all the methods described herein can be performed in any appropriate order. Unless otherwise stated herein, all the examples or exemplary expressions used herein (for example, "for example") are only intended to simply, better describe the present invention and are not for providing limitations to the scope of the present invention. Note that none of the expressions in the specification shall be construed as indicating an element that is not described in the claims as essential for the present invention to be carried out.

Preferred embodiments of the present invention including a best mode that the present inventors know to carry out the present invention are described herein. For those skilled in the art, modifications of these preferred embodiments will be apparent by reading the description above. The present inventors expect that experts will apply these modifications as appropriate, and anticipate that the present invention will be carried out by methods other than the methods specifically described herein. Thus, the present invention includes, as permitted by the governing law, all the revisions made to and equivalents to the content described in the claims attached to the present specification. Furthermore, unless otherwise indicated herein or clearly contradicted with the context, the present invention also includes all combinations of the elements in all the modifications.

### Reference Signs List

- 1: image processing apparatus (ultrasound diagnostic apparatus)
- 2: measurement table
- 3: measurement apparatus
- 10: bath
- 20: ring array (measurement device)
- 21: transducer base
- 22: transducer
- 24: resin mold
- 30: collection tank
- 33: overflow detection sensor (upper drainage detection sensor)
- 40: top cover
- 42: top side opening (opening)
- 44: gap
- 50: lifter
- 55: accordion portion (liquid-tight cover)
- 70: storage tank
- 80: deaeration module
- 100: control box
- 110: image control unit
- 120: mechanical control unit
- 123: liquid level control module
- 130: database
- 140: PC
- 150: user interface
- 160: PACS
- CS: circulation system
- F: hollow fiber filter (filter)
- P: examinee
- S: test object
- W: propagation liquid

## Claims

1. An image processing apparatus comprising:
a bath an inside of which is filled with a propagation liquid and in which a test object is to be immersed;
a measurement device having a group of elements that irradiates the inside of the bath with a radiation wave and receives the radiation wave, which is a scattered wave;
an upper drainage detection sensor that detects draining of the propagation liquid from a top portion of the bath to outside the bath; and
a liquid level control module that controls a liquid level of the propagation liquid in the inside of the bath, wherein
the liquid level control module controls, before the test object is immersed in the inside of the bath, an amount of the propagation liquid in the inside of the bath such that the liquid level becomes lower than a top end position of the bath, and performs control, after the test object is immersed in the inside of the bath, such that the liquid level is increased by supplying the propagation liquid into the inside of the bath at least until the upper drainage detection sensor detects draining of the propagation liquid.

2. The image processing apparatus according to Claim 1, further comprising:
a collection tank that is disposed at or near an outer periphery of the bath and collects the propagation liquid drained from the top portion of the bath to outside the bath, wherein the upper drainage detection sensor is disposed at the collection tank.

3. The image processing apparatus according to Claim 1 or 2,
wherein a top cover having, at a center portion thereof, an opening through which the test object is insertable is disposed above the bath, and a predetermined gap is formed between a top end portion of the bath and an undersurface of the top cover.

4. The image processing apparatus according to Claim 3,
wherein the opening of the top cover is smaller than a top opening of the bath and positioned inside the top opening of the bath in a plan view, and a region surrounding the opening of the top cover forms a tapered surface inclined downward.

5. The image processing apparatus according to Claim 3 or 4,
wherein the top end portion of the bath is positioned lower than the opening of the top cover in height.

6. The image processing apparatus according to any one of Claims 2 to 5, further comprising:
a circulation system for circulating the propagation liquid in the inside of the bath, and
the circulation system includes:
a storage tank that stores the propagation liquid;
a propagation liquid supply module for supplying the propagation liquid in an inside of the storage tank to the bath;
a collection-tank side drain module that returns the propagation liquid collected by the collection tank to the storage tank;
a bath side drain module that is provided at the bottom portion of the bath and returns the propagation liquid in the inside of the bath to the storage tank;
a filter that purifies the propagation liquid to be supplied to the bath; and
a deaeration module that removes air in the propagation liquid to be supplied to the bath.

7. The image processing apparatus according to Claim 6,
wherein the propagation liquid supply module includes a propagation liquid supply valve that controls supply of the propagation liquid, and the propagation liquid supply valve has a supply amount adjustment structure that enables maintaining of supply of a predetermined amount of the propagation liquid while the measurement device is in operation.

8. An image processing apparatus control program for causing an image processing apparatus to execute an operation below, the image processing apparatus including a bath an inside of which is filled with a propagation liquid and in which a test object is to be immersed, a measurement device having a group of elements that irradiates the inside of the bath with a radiation wave and receives the radiation wave, which is a scattered wave, and an upper drainage detection sensor that detects draining of the propagation liquid from a top portion of the bath to outside the bath, the operation comprising:
(1) a step for filling the inside of the bath with the propagation liquid an amount of which is smaller than a capacity of the bath by a predetermined amount before the test object is immersed in the inside of the bath;
(2) a step for supplying the propagation liquid into the inside of the bath;
(3) a step for detecting draining of the propagation liquid using the upper drainage detection sensor; and
(4) a step for allowing the measurement device to perform measurement in a case where draining of the propagation liquid is detected.

9. The image processing apparatus control program according to Claim 8, after the (3), the operation further comprising:
(5) a step for reducing an amount of the propagation liquid to be supplied into the inside of the bath per unit time or stopping supply of the propagation liquid.

10. The image processing apparatus control program according to Claim 8 or 9, after the (4) and in a case where the test object is moved to outside the bath, the operation further comprising:
(6) a step for supplying the propagation liquid into the inside of the bath to drain the propagation liquid in the inside of the bath from a top portion of the bath to outside the bath and/or for draining a predetermined amount of the propagation liquid in the inside of the bath from a bottom portion of the bath.

11. The image processing apparatus control program according to any one of Claims 8 to 10,
wherein the predetermined amount in the (1) is step-wise adjustable.
